# EUROPEAN PATENT SPECIFICATION

(11) **EP 0 983 040 B1**
(45) Date of publication and mention of the grant of the patent: **15.01.2003**
(21) Application number: 98920886.3
(22) Date of filing: 13.05.1998
(51) Int. Cl.: A61K 7/06

(54) **HAIR FIXING COMPOSITION**
HAARFESTIGUNGSMITTEL
COMPOSITION DE LAQUE CAPILLAIRE

(30) Priority: 23.05.1997 US 862628
(43) Date of publication of application: 08.03.2000
(73) Proprietor: Colgate-Palmolive Company (a Delaware corporation), New York, N.Y. 10022 (US)
(72) Inventor: HARDY, Eugene, E., 08857 New Jersey (US); PSIHOULES, Anthony, Somerville, NJ 08876 (US)
(74) Representative: Ottevangers, Sietse Ulbe
(86) International application number: US9808425
(87) International publication number: WO98052517

(56) References cited:
- EP-A- 0 418 676
- DE-A- 4 239 499
- FR-A- 2 684 000

## Description

### BACKGROUND OF THE INVENTION

Hair fixing compositions usually comprise solutions or suspensions with film forming natural or synthetic polymers. Numerous polymers have been used in hair fixing formulations such as natural materials as gums, shellacs, chitosan, and protein derivatives or synthetic polymers with specific applications in hair fixing technology such as polyvinylpyrrolidones, copolymers of polyvinylpyrrolidone-vinyl acetate, acrylates, acrylate acrylamides, crotonic acid containing copolymers usually substantially neutralized with a basic component. Examples of such crotonic acid polymers include neutralized crotonic acid/vinylacetate, and crotonic acid/vinylacetate, vinylpropionate and the like. Combinations of various polymers are now known as the active components in hair fixative formulations such as polyvinylpyrrolidone vinylacetate (Luviskol®) with neutralized crotonic acid/vinylacetate/vinylpropionate, Luviset® CAP in unneutralized form, both polymers manufactured by BASF, see WO 94/12147 and Luviskol® with Amphomer, an amphoteric resin of octylacrylamide/acrylates/butylaminoethyl methacrylate copolymer manufactured by National Starch, see DE 4,034,315 Al.

Additionally DE 4,239,499 Al discloses combination of various polymers for hair styling. A neutralized vinylacetate/crotonic acid/vinylpropionate terpolymer is employed. EP 0 418 676 A and FR 2684000 both relate to hair fixing compositions that may contin one or more polymers selected from a list comprising vinylpyrrolidone/vinylacetate copolymer, neutralized vinylacetate crotonic acid vinylneodecanoate terpolymer and further polymers. However, neither of the disclosures specifically disclose the combination of the said two polymers.

The polyvinylpyrrolidone/vinyl acetate hereafter referred to as "PVP/VA" copolymers have been used in several dual resin compositions. By themselves, they have been successfully marketed as a single resin system for years for their overall grouping of properties, but the major property of a hair fixative, namely its "hold" property can be improved. Other synthetic polymers have better "hold" properties but lack other attributes which would bring about a high quality hair fixative formulation.

It has now been discovered that a copolymer of PVP/VA can be successfully combined with a copolymer comprising vinyl acetate, neutralized crotonic acid, and vinylneodecanoate, obtained as Resyn 28-2930 or 28-2942 without neutralization from National Starch, and obtain a highly desirable and surprising mix of properties, which are substantially better than a combination of PVP/VA (Luviskol®) with a copolymer of neutralized vinyl acetate, crotonic acid and vinylpropionate (Luviset® CAP), which is a structurally closely related combination of polymers to the invention combination of polymers.

### SUMMARY OF THE INVENTION

In accordance with the invention there is a hair fixing composition comprising
a. from about 0.5 to about 10 wt. % of a copolymer of PVP/VA,
b. from about 0.5 to about 8 wt. % of at least about 80 wt. % neutralized copolymer of vinyl acetate, crotonic acid, and vinylneodecanoate, and
c. a solvent system compatible with the combination of copolymer a and copolymer b the wt. % of a to b, in the range about 1 to about 5 and about 5 to about 1.

It should be noted that it is possible there can be chemical reactions between and among the components listed above and any other component which may be present in the composition. Therefore, it is intended to include such reaction product within the claimed composition as well.

### DETAILED DESCRIPTION OF THE INVENTION

The copolymer comprising PVP/VA is obtained from BASF as Luviskol®. The amount of each monomer in the polymer is preferably from about 20 to about 50 wt. % vinylpyrrolidone (VP) and about 80 to about 50 wt. % vinylacetate (VA), more preferably about 25 to 40 wt. % VP and about 75 to about 60 wt. % VA. Especially preferred is the ratio of about 30 wt. % VP and about 70 wt. % VA available commercially from BASF as Luviskol® 37I, a 50 wt. % polymer solution in isopropanol. The PVP/VA copolymer is preferably in the hair composition at quantities of from about 1 to about 6 wt. %.

The second copolymer comprises a polymer formed from vinyl acrylate, crotonic acid and vinylneodecanoate. The polymer is generally at least about 80% neutralized, preferably at least about 90% neutralized, and can be 100% neutralized. Any basic neutralizing agent compatible with the composition can be employed even inorganic materials such as sodium or potassium hydroxide. Generally organic amines or alkanolamines are readily used for neutralization. As aforestated the polymer can be obtained in unneutralized form from National Starch Company as Resyn 28-2930 or 28-2942.

Examples of such neutralizing amines include:
triethanolamine
2-amino-2-methyl-1,3-propandiol
2-amino-2-ethyl-1,3-propanediol
2-amino-2-methyl- 1-propanol
2-amino-2-methyl-2-pentanol
1-amino-2-methyl-2-propanol
2-amino-1-butanol
3-amino-2-pentanol
2-amino-1-phenyl-1-butanol
2-dimethylamino-2-methyl-1-propanol
2-dimethylamino-2-methyl-1,3-propandiol
tris-(hydroxymethyl)-aminomethane
tris-(hydroxymethyl)-dimethylaminamethane
N¹-(2-hydroxyethyl)-2-methyl-1,2-propandiamine
Neutralization with 2-amino-2-methyl-1-propanol or 2-amino-2-methyl-1-3-propandiol or triethanolamine is preferred.

The quantity of partially or completely neutralized crotonic acid copolymer present in the composition is preferably about 1 to about 6 wt. %. Preferably polymer a and polymer b are present in the composition in a weight ratio of about 1:3 to about 3:1 polymer a to polymer b.

The vehicles which carry the polymers are generally alcoholic or hydroalcoholic. The alcohol contained in the hair fixing composition is one of the common lower alcohols, such as isopropanol and ethanol, typically used in similar types of hair fixing compositions. The alcohol can be used in amounts between about 10% to about 90% by weight of the composition, preferably about 15% to about 85%.

The hair fixing composition can contain a total water content of 0 up to about 20% by weight, a water content of about 0.5 to about 15% by weight being preferred.

The hair fixing composition can have pH values of between 4.5-9.5.

The composition can also contain other typical cosmetic ingredients such as perfume, oils, extracts, vitamins and their derivatives/precursors, plasticizing agents. hydrophobing substances such as silicone derivatives, solubilizers for oils, and preservatives up to 1 % each by weight if found to be advantageous to the composition.

The hair fixing composition can be filled into pressurizable containers, preferably with a propellant added or utilized as a non-propellant hair styling product.

When the composition is utilized as an aerosol hair fixing product, it contains an additional about 5 to about 75% by weight of the total composition, preferably about 25% to about 70% by weight of a propellant and is filled into an appropriate pressurizable container.

Suitable propellants for the hair fixing composition include the lower alkanes such as n-butane, iso-butane, propane and mixtures thereof with fluorocarbon 152a and dimethylether as well as compressed gases such as N₂, N₂O, CO₂ and mixtures thereof with the above-mentioned propellants.

When the composition is present as a non-aerosol hairspray or concentrated hairspray, the composition can be sprayed by utilizing a satisfactory mechanically operated spray pump device.

A mechanically operated spray pump device allows a liquid to be sprayed without use of propellants as listed above. An example, but not limited to that is a spray pump or an elastic container equipped with an attached spraying valve in which the hair fixing composition is filled under high pressure expanding the container and in which the composition is actuated upon opening the spraying valve can be used as a suitable spraying device/package.

The hair fixing composition is excellently compatible with halogen-free hydrocarbons and/or dimethyl ether and results in outstanding hair fixing properties.

The following examples serve to explain the subject matter of the composition more fully and are not intended to unduly narrow the invention. In the text below VP is the abbreviation for vinylpyrrolidone and VA is the abbreviation for vinyl acetate. PVP is polyvinyl pyrrolidone. Resyn 28-2930 has been previously identified as vinylacetate, crotonic acid, vinylneodecanoate and Luviset® CAP has been previously identified as a copolymer of vinylacetate, vinylpropionate and crotonic acid. Both the Resyn 28-2930 and the Luviset® CAP are purchased in unneutralized form and are neutralized by an amine. The PVP/VA employed in the examples is 30 wt. % PVP, 70 wt. % VA.

### EXAMPLES

### Example 1

The following hair spray formulations are compared for curl retention using the following method. In a standard high humidity curl retention test the hairspray of the invention, Example 1, is compared to the prior art, control. Specifically, 3 gram 8" long virgin brown European hair tresses freshly shampooed with 10% sodium laureth (2EO) sulfate solution and treated with a 3-second hair spray application are rolled onto a 5/8" diameter roller and fastened. The rolled tresses are allowed to dry in a closed cabinet with a hair dryer (at low heat setting) for 30 minutes to simulate salon drying conditions.

The tresses are unrolled on a curl retention board which contains measurement lines where initial curl length readings are taken. The curl retention boards are subsequently placed in an 85% humidity chamber maintained at a temperature of about 21.6C for a period of 90 minutes. Typically, eight tresses are evaluated for each treatment. Readings are taken at 15 minute intervals. Results are evaluated statistically. The results stated in Table 1 represent the average evaluation for each treatment.

| | **Examule 1** **(PVP/VA & Resyn 28-2930)** | **Control** **(PVP/VA & Luviset® CAP)** |
|---|---|---|
| Denatured Alcohol | 37.35 | 37.39 |
| PVP/VA 50% in isopropanol | 2.00 | 2.00 |
| Luviset® CAP | --- | 3.75 |
| Resyn 28-2930 | 3.75 | --- |
| Phenyl Trimethicone | 0.25 | 0.25 |
| 2-Ammo-2-methyl-1-propanol (AMP-95) | 0.35 | 0.31 |
| Fragrance | 0.3 | 0.3 |
| N-butane | 22.4 | 22.4 |
| Dimethy Ether | 33.6 | 33.6 |

### Below are the results

Average % Curl Retention from initial Time

| Time | | | | | | | |
|---|---|---|---|---|---|---|---|
| | Initial | 15' | 30' | 45' | 60' | 75' | 90' |
| Control | 100 | 78.6 | 67.9 | 61.2 | 53.4 | 45.7 | 42.7 |
| Example 1 | 100 | 85.4 | 80.7 | 76.4 | 71.6 | 67.3 | 63.7 |

From this data it is clear that the composition of the invention provides far better curl retention than the structurally closely related composition of the prior art wherein one polymer is the same as that of the invention composition and the second polymer of the prior art compositions has two monomers the same as the invention's second polymer and the third monomer being vinyl propionate as opposed to vinyl neodecanoate of the invention composition.

### Example 2

Approximately 100 women use each of the two hair sprays described below, one an example of the invention and the second a control for three weeks, utilizing the hair spray at least twice each week. The following formulations were compared (Quantities are in weight percent).

| | **Example 2 (Luviskol® & Resvn 28-2930)** | **Control (Luviskol® & Luviset® CAP) (as Analyzed)** |
|---|---|---|
| Denatured Alcohol | 31.7 | 31.7 |
| PVP/VA 50% in isopropanol | 8.0 | 7.8 |
| Resyn 28-2930 | 3.1 | --- |
| Luviset® CAP | --- | 3.0 |
| 2-Amino-2-methyl-1-propanol | 0.3 | 0.3 |
| Other | 1 | 1 |
| Dimethylether/N-butane mixture | 56 | 56 |

The composition of the invention, Example 2, is found to be better than the control at at least a 90% confidence level for the following qualities and parameters. Those qualities marked with an asterisk were found to be better at a 95% confidence level.
Buying intention*
Gives hairstyle a natural hold*
Gives hair elastic hold*
Gives hairstyle a good hold*
Hairstyle stays manageable after applying hairspray*
Gives hair volume
Easily washed out*
Hair less dry and brittle
Less stickiness
Less residue on hair*

### Example 3

The compositions of Example 2 are applied to ten mannequin heads in sequence and the characteristics evaluated by a trained cosmetologist who is blind to the nature of the compositions. The evaluations are consistent with the results of Example 2.

The compositions of the invention are substantially and surprisingly better than the prior art composition in numerous hair care properties.

## Claims

1. A hair fixing composition comprising
a) from about 0.5 to about 10 wt. % of a copolymer of polyvinylpyrrolidone and vinylacetate,
b) from about 0.5 to about 8 wt. % of at least about 80 wt. % neutralized copolymer of vinyl acetate, crotonic acid and vinylneodecanoate, and
c) a solvent system compatible with the combination of copolymer a and copolymer b, the wt. % of a to b in the range of about 1:5 to about 5:1.

2. The composition in accordance with claim 1 wherein copolymer a has from about 20 to about 50 wt. % vinylpyrrolidone and about 80 to about 50 wt. % vinyl acetate.

3. The composition in accordance with claim 2 wherein the vinylpyrrolidone is from about 25 to about 40 wt. % and the vinyl acetate is from about 75 to about 60 wt. %.

4. The composition in accordance with claim 1 wherein copolymer a is present in the composition in quantities of about 1 to about 6 wt. %.

5. The composition in accordance with claim 1 wherein copolymer b is at least about 90 wt. % neutralized.

6. The composition in accordance with claim 1 wherein copolymer b is present in the composition in quantities of from about 1 to about 6 wt. %.

7. The composition in accordance with claim 1 wherein alcohol or a combination of alcohol and water is present

8. The composition in accordance with claim 7 wherein alcohol is about 10 to about 90 wt. % of the composition.

9. The composition in accordance with claim 8 wherein water is zero to about 20 wt.% of the composition.

10. The composition in accordance with claim 1 wherein the pH of the composition is between about 4.5 and about 9.5.

11. The composition in accordance with claim 1 which is an aerosol and has a propellant therein.

12. The composition in accordance with claim 11 wherein the propellant is about 5 to about 75 wt. % of the total composition.

13. A product which comprises the composition obtainable by contacting in any order
a) from about 0.5 to about 10 wt. % of a copolymer of polyvinylpyrrolidone and vinylacetate,
b) from about 0.5 to about 8 wt. % of at least about 80 wt. % neutralized copolymer of vinyl acetate, crotonic acid and vinylneodecanoate, and
c) a solvent system compatible with the combination of copolymer a and copolymer b, the wt. % of a to b in the range of about 1:5 to about 5:1.

## Patentansprüche

1. Haarfestigungszusammensetzung, die
a) etwa 0,5 bis etwa 10 Gew.-% Copolymer aus Polyvinylpyrrolidon und Vinylacetat,
b) etwa 0,5 bis etwa 8 Gew.-% mindestens etwa 80 Gew.-% neutralisiertes Copolymer aus Vinylacetat, Crotonsäure und Vinylneodecanoat und
c) ein Lösungsmittelsystem umfasst, das mit der Kombination aus Copolymer a und Copolymer b verträglich ist, wobei das Gewichtsverhältnis von a zu b im Bereich von etwa 1 : 5 bis etwa 5 : 1 liegt.

2. Zusammensetzung nach Anspruch 1, bei der Copolymer a etwa 20 bis etwa 50 Gew.-% Vinylpyrrolidon und etwa 80 bis etwa 50 Gew.-% Vinylacetat aufweist.

3. Zusammensetzung nach Anspruch 2, bei der das Vinylpyrrolidon etwa 25 bis etwa 40 Gew.-% ausmacht und das Vinylacetat etwa 75 bis etwa 60 Gew.-% ausmacht.

4. Zusammensetzung nach Anspruch 1, bei der Copolymer a in der Zusammensetzung in Mengen von etwa 1 bis etwa 6 Gew.-% vorhanden ist.

5. Zusammensetzung nach Anspruch 1, bei der Copolymer b mindestens etwa 90 Gew.-% neutralisiert ist.

6. Zusammensetzung nach Anspruch 1, bei der Copolymer b in der Zusammensetzung in Mengen von etwa 1 bis etwa 6 Gew.-% vorhanden ist.

7. Zusammensetzung nach Anspruch 1, bei der Alkohol oder eine Kombination von Alkohol und Wasser vorhanden ist.

8. Zusammensetzung nach Anspruch 7, bei der Alkohol etwa 10 bis etwa 90 Gew.-% der Zusammensetzung ausmacht.

9. Zusammensetzung nach Anspruch 8, bei der Wasser 0 bis etwa 20 Gew.-% der Zusammensetzung ausmacht.

10. Zusammensetzung nach Anspruch 1, bei der der pH-Wert der Zusammensetzung zwischen etwa 4,5 und etwa 9,5 liegt.

11. Zusammensetzung nach Anspruch 1, die ein Aerosol ist und ein Treibmittel enthält.

12. Zusammensetzung nach Anspruch 11, bei der das Treibmittel etwa 5 bis etwa 75 Gew.-% der Zusammensetzung ausmacht.

13. Produkt, das eine Zusammensetzung umfasst, die durch Kontaktierung von
a) etwa 0,5 bis etwa 10 Gew.-% Copolymer aus Polyvinylpyrrolidon und Vinylacetat,
b) etwa 0,5 bis etwa 8 Gew.-% mindestens etwa 80 Gew.-% neutralisiertem Copolymer von Vinylacetat, Crotonsäure und Vinylneodecanoat und
c) einem Lösungsmittelsystem, das mit der Kombination aus Copolymer a und Copolymer b verträglich ist, wobei das Gewichtsverhältnis von a zu b im Bereich von etwa 1 : 5 bis etwa 5 : 1 liegt, in irgendeiner Reihenfolge erhältlich ist.

## Revendications

1. Composition de laque capillaire comprenant :
a) d'environ 0,5 à environ 10% en poids d'un copolymère de polyvinylpyrrolidone et d'acétate de vinyle,
b) d'environ 0,5 à environ 8% en poids d'au mois environ 80% en poids de copolymère neutralisé d'acétate de vinyle, d'acide crotonique et de néodécanoate de vinyle, et
c) un système de solvant compatible avec la combinaison du copolymère a et du copolymère b, le % en poids de a à b se situant dans la plage d'environ 1:5 à environ 5:1.

2. Composition selon la revendication 1, dans laquelle le copolymère a d'environ 20 à environ 50% en poids de vinylpyrrolidone, et d'environ 80 à environ 50% en poids d'acétate de vinyle.

3. Composition selon la revendication 2, dans laquelle la vinylpyrrolidone est comprise d'environ 25 à environ 40% en poids et l'acétate de vinyle est compris d'environ 75 à environ 60% en poids.

4. Composition selon la revendication 1, dans laquelle le copolymère a est présent dans la composition en quantités d'environ 1 à environ 6% en poids.

5. Composition selon la revendication 1, dans laquelle le copolymère b est neutralisé au moins à environ 90% en poids.

6. Composition selon la revendication 1, dans laquelle le copolymère b est présent dans la composition en quantités d'environ 1 à environ 6% en poids.

7. Composition selon la revendication 1, dans laquelle l'alcool ou une combinaison d'alcool et d'eau est présente.

8. Composition selon la revendication 7, dans laquelle l'alcool est compris d'environ 10 à environ 90% en poids de la composition.

9. Composition selon la revendication 8, dans laquelle l'eau est comprise de zéro à environ 20% en poids de la composition.

10. Composition selon la revendication 1, dans laquelle le pH de la composition a une valeur d'environ 4,5 à environ 9,5.

11. Composition selon la revendication 1, sous forme d'aérosol et contenant un agent de propulsion.

12. Composition selon la revendication 11, dans laquelle l'agent de propulsion est compris d'environ 5 à environ 75% en poids de la composition totale.

13. Produit comprenant la composition susceptible d'être obtenue en mettant en contact dans un ordre quelconque
a) d'environ 0,5 à environ 10% en poids d'un copolymère de polyvinylpyrrolidone et d'acétate de vinyle.
b) d'environ 0,5 à environ 8% en poids d'au moins environ 80% en poids de copolymère neutralisé d'acétate de vinyle, d'acide crotonique et de néodécanoate de vinyle, et
c) un système de solvant compatible avec la combinaison du copolymère a et du copolymère b, le % en poids de a à b se situant dans la plage d'environ 1:5 à environ 5:1.
